# EUROPEAN PATENT APPLICATION

(11) **EP 3 144 321 A1**
(43) Date of publication of application: **22.03.2017**
(21) Application number: 15185930.3
(22) Date of filing: 18.09.2015
(51) Int. Cl.: C07K 16/18, C12N 5/16, G01N 33/68, C12N 15/85

(54) **MAMMALIAN DICKKOPF 3 (DKK3) AS URINARY MARKER FOR RENAL INTERSTITIAL FIBROSIS/TUBULAR ATROPHY (IF/TA) AND PROGRESSIVE KIDNEY FAILURE**

(71) Applicant: Deutsches Krebsforschungszentrum Stiftung des Öffentlichen Rechts, 69120 Heidelberg (DE)
(72) Inventor: Gröne, Hermann-Josef, 69118 Heidelberg (DE); Arnold, Bernd, 69124 Heidelberg (DE)
(74) Representative: Krauss, Jan

(57) **Abstract**

The present invention relates to mammalian Dickkopf 3 (Dkk3) as a marker of renal interstitial fibrosis/tubular atrophy (IF/TA), assessing the extent of renal tissue damage and thereby the risk of progression of chronic kidney disease (CKD), and the effect of therapeutic interventions on the progression of IF/TA in CKD, as well as to related methods and uses. In particular, the present invention relates to Dkk3 monoclonal antibodies, as well as fragments, regions and derivatives thereof, and an improved method for diagnosing renal IF/TA, and progressive CKD using said antibodies.

## Description

The present invention relates to mammalian Dickkopf 3 (Dkk3) as a marker of renal interstitial fibrosis/tubular atrophy (IF/TA), assessing the extent of renal tissue damage and thereby the risk of progression of chronic kidney disease (CKD), and the effect of therapeutic interventions on the progression of IF/TA in CKD, as well as to related methods and uses. In particular, the present invention relates to Dkk3 monoclonal antibodies, as well as fragments, regions and derivatives thereof, and an improved method for diagnosing renal IF/TA, and progressive CKD using said antibodies.

### Background of the present invention

Chronic kidney disease (CKD) causes a progressive and permanent loss of kidney function, finally necessitating permanent renal replacement therapy when uremia becomes life threatening. CKD must be separated from acute kidney injury, the latter being characterized by rapid loss of kidney function within days, which is, however, usually reversible.

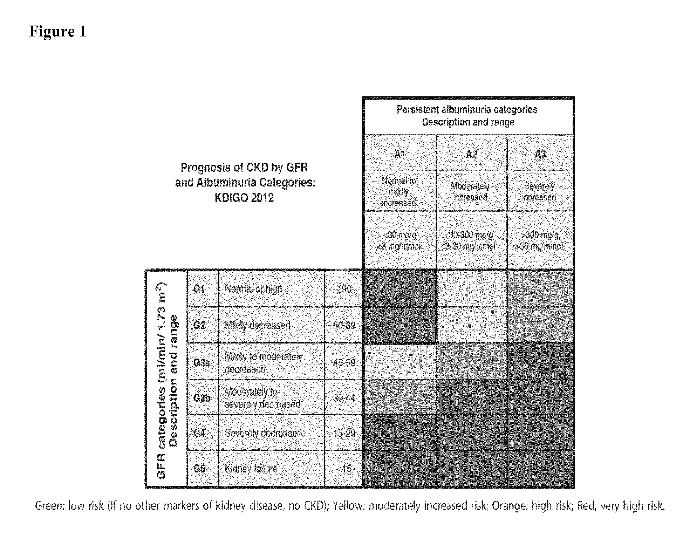

The international Kidney Disease Improving Global Outcomes (KDIGO) 2012 Clinical Practice Guideline for the Evaluation and Management of Chronic Kidney Disease defines CKD as abnormalities of kidney structure or function, present for >3 months, with implications for health. Moreover, the guideline also classifies CKD into different stages based on kidney function, i.e. glomerular filtration rate (GFR), and albumin excretion in the urine. The CKD stages cover a wide range of kidney function from normal GFR to the establishment of permanent kidney failure, with a final reduction of GFR to below 15% of normal. At this stage, the patient is considered to suffer from end stage renal disease, which sooner or later needs renal replacement therapy. The CKD stages are accompanied by different degrees of albuminuria, and that the risk of progression of CKD is deduced from two variables: GFR and urine albumin excretion (Figure 1).

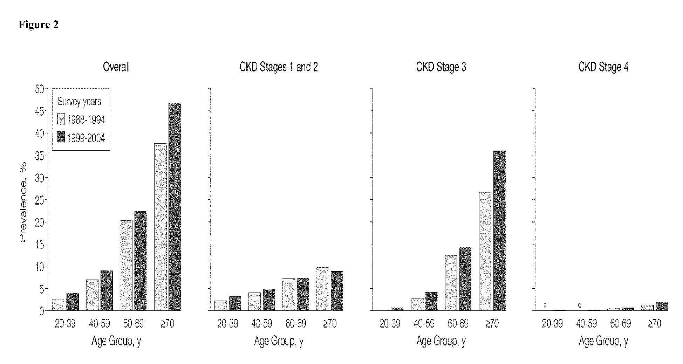

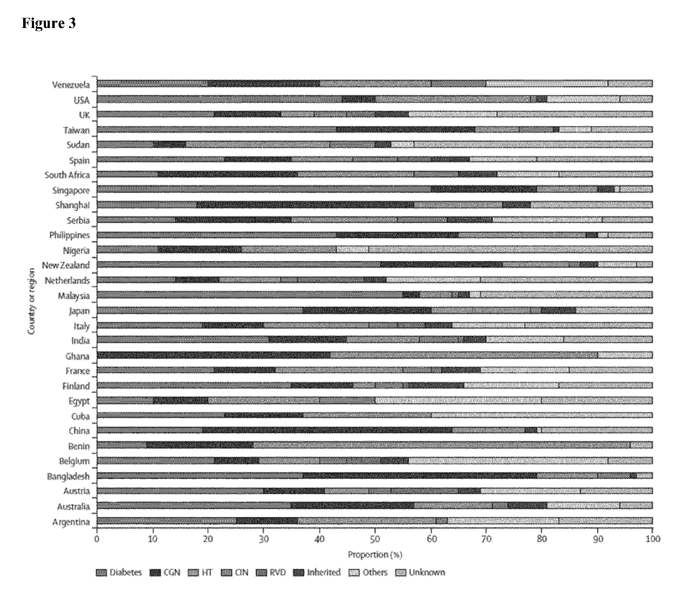

Over the last decades, both incidence and prevalence rates of all CKD stages increased substantially worldwide, but the increase was particularly evident in the more advanced CKD stages 3 and 4. This is best documented by data from large U.S. epidemiological studies such as the Health and Nutrition Examination Survey (NHANES) (Figure 2). The steady growth in the incidence and prevalence of CKD is mainly a result of ageing populations with an increase in diseases that lead to kidney injury, such as diabetes, hypertension and renovascular disease. These three conditions account for far more than 50% of progressive CKD not only in the Western societies, but meanwhile also in the developing countries (Figure 3). Because of the considerable treatment costs for patients with failing kidneys - especially those requiring renal replacement therapy - early detection and adequate treatment of progressive CKD is of paramount importance from a medico-economical standpoint of view.

Monitoring of kidney function and thus also of CKD progression is usually performed with the measurement of surrogate markers of GFR, mostly the concentration of creatinine in blood. Creatinine is the break-down product of skeletal muscles, which passes the glomerular filtration barrier and is freely filtered in the urine. Since it is virtually not reabsorbed in the renal tubular system, its concentration in blood depends almost exclusively on the GFR. In CKD the affected kidneys do no longer filtrate creatinine properly, resulting in its accumulation in blood and other body fluids. However, because of the exponential relationship between the decline of GFR and rise of creatinine blood levels, this surrogate marker provides only an approximation of kidney function. In children and in elderly, creatinine is even more insufficient in this respect because of their low muscle mass. In clinical practice, an attempt to bypass the inaccuracy of creatinine blood measurements is the calculation of estimated glomerular filtration rate (eGFR) using different mathematical equations that are based on age, race, gender, and blood creatinine concentration. Nevertheless, these estimates of kidney function suffer from the inherent problem that they reflect only the GFR - a physiological variable-rather than the real extent of whole kidney tissue damage.

In this respect, the assessment of the amount of proteins in urine, such as albumin, is a more reliable sign of definite kidney injury, since the finding of pathological amounts of proteins in urine results from both, glomerular filtration barrier damage as well as injury to the renal tubulo-interstitium. According to the KDIGO 2012 Clinical Practice Guideline for the Evaluation and Management of Chronic Kidney Disease, testing for albumin in urine is therefore used for the detection and classification of chronic kidney injury, and the risk assessment for progressive CKD (Figure 1). Dipstick tests made with a color-sensitive pad are used to indicate the level of albumin in the urine, but they are semi-quantitative and give only rough estimates of the albumin urine content. As for the measurement of creatinine in blood, the correlation of albuminuria to renal tissue damage is rather modest. In addition, small amounts of albumin in the urine are usually not detected when a routine dipstick test is performed.

Taken together, definite confirmation of progressive and destructive CKD can be obtained only by kidney biopsy, i.e. an invasive procedure that provides renal tissue samples for histopathological examination. Here, the finding of renal tubulo-interstitial damage is a hallmark of serious tissue damage, and thus progressive CKD. Specifically, renal IF/TA is the final common pathway of etiologically different progressive CKD, which inevitably result in organ failure. Unfortunately, suitable non-invasive diagnostic tests for this decisive pathologic manifestation of progressive CKD are not available to date.

During the past decade, the application of gene expression profiling in cancer research has resulted in development of new therapeutic targets. In contrast to the success of gene expression profiling in oncology, several challenges have severely limited the application of genomic profiling in non-malignant kidney diseases. First, tissue availability is limited because diagnostic kidney biopsies or non-malignant nephrectomies are performed relatively infrequently. Second, the composition of kidney tissue specimens is inherently heterogeneous contributing to sampling error which renders standardized, quantitative gene expression profiling across large series of kidney biopsies technically challenging.

PCT/EP2015/056975 relates to a method for diagnosing chronic kidney disease, comprising the steps of a) obtaining a urine sample comprising early morning urine (EMU) from a mammalian, preferably a human, patient to be diagnosed, b) measuring the amount of Dickkopf 3 (DKK3) protein and/or adducts thereof in said sample, and c) concluding on a CKD of said patient, wherein a higher amount of DKK3 protein and/or adducts thereof in said sample compared to a healthy patient is indicative for a chronic kidney disease.

Assays that use antibodies for a detection of DKK3 are commercially available, nevertheless, these assays have, for example, recovery rates in pre-conditioned samples of 86.69% in serum and 89.24% in plasma (i.e. between 10 and 15% divergence in the test). With urine samples, it was found that usually an additional optimization is required, rendering these tests difficult to use in a standardized format.

Thus, one of the most important unmet needs in renal medicine is the identification and validation of efficient tools and assays for markers for CKD, and in particular renal IF/TA, and the progression of CKD that facilitate targeted treatment of those individuals at high risk, while avoiding unnecessary treatment of others. Other objects and advantages of the present invention will become apparent to the person of skill when studying the specification of the present invention.

In a first aspect thereof, the object of the present invention is solved by providing a monoclonal antibody specific for Dickkopf 3 (Dkk3) protein, comprising at least one light chain and at least one heavy chain, wherein said at least one light chain comprises a polypeptide comprising an amino acid sequence having at least 90% identity with a light chain variable region of a monoclonal antibody and wherein said at least one heavy chain comprises a polypeptide comprising an amino acid sequence having at least 90% identity with a heavy chain variable region of a monoclonal antibody, wherein said monoclonal antibody is produced by the hybridoma cell line huDkk3-16.1, huDkk3-2.13 or huDkk3-24.20 as deposited under the Budapest Treaty on September 8, 2015 at the DSMZ in Braunschweig, Germany under deposit number DSM ACC3274, DSM ACC3275, and DSM ACC3276, respectively.

The antibodies of the invention are specifically reactive with epitopes on the Dickkopf 3 (Dkk3) protein, meaning that they selectively recognize and bind to said epitopes. Any conventional binding assay can be used to assess this binding, including for example, an enzyme linked immunosorbent assay.

The peptides of the invention, including antibodies and antibody fragments, have particular utility in the detection of Dkk3 protein, and the diagnosis, monitoring and/or prognosis of progressive and destructive CKD, such as renal IF/TA.

In one embodiment, the isolated antibody or antibody fragment according to the present invention may be an isolated intact soluble monoclonal antibody. The isolated antibody or antibody fragment may be an isolated monoclonal antibody fragment selected from the group consisting of a Fab, Fab', F(ab')2, Fv, SFv, or scFv. These fragments are produced from intact antibodies using methods well known in the art such as, for example, proteolytic cleavage with enzymes such as papain (to produce Fab fragments) or pepsin (to produce F(ab')2). Preferred is the monoclonal antibody according to the present invention, wherein the percentages identity are at least 90%, more preferably at least 95%, such as, for example, 98%. Most preferably, the variable regions of the monoclonal antibody according to the present invention consist of an amino acid sequence according to a monoclonal antibody, wherein said monoclonal antibody is produced by the hybridoma cell line huDkk3-16.1, huDkk3-2.13 or huDkk3-24.20 as deposited under the Budapest Treaty on September 8, 2015 at the DSMZ in Braunschweig, Germany under deposit number DSM ACC3274, DSM ACC3275, and DSM ACC3276, respectively.

Included in the invention are also antibodies, wherein the variable regions of said antibody are modified because of post-translational modifications. These modifications include, for example, incomplete disulfide bond formation, glycosylation, N-terminal pyroglutamine cyclization, C-terminal lysine processing, deamidation, isomerization, and oxidation, and less common ones such as modification of the N-terminal amino acids by maleuric acid and amidation of the C-terminal amino acid. Modifications can be introduced in vitro and/or in vivo. Of course, according to the invention, these modified antibodies still bind specifically to dkk3 epitope(s).

The antibodies of the present invention exhibit very strong binding (and thus have a high affinity), i.e., O.D.s of around twice background in an enzyme-linked immunosorbent assay against a test antibody. In a preferred embodiment, the level of high binding is equal to or greater than five times background. In other embodiments, the level of high binding is equal to or greater than 10 times background. Of course, any meaningful increase over background (the level observed when all the reagents other than the antibody being tested) will be recognized by skilled persons in the art as high binding and therefore within the scope of the invention.

In a particularly preferred aspect of the invention, the antibody is a recombinant human antibody made up of regions from the antibodies of the invention together with constant regions of human antibodies (IgG). For example, an H chain can comprise the antigen-binding region of the heavy chain variable region of an antibody of the invention linked to at least a portion of a human heavy chain constant region. This humanized or chimeric heavy chain may be combined with a chimeric L chain that comprises the antigen binding region of the light chain variable region of the antibody linked to at least a portion of the human light chain constant region.

The recombinant antibodies of the invention may be monovalent, divalent, or polyvalent immunoglobulins. For example, a monovalent antibody is a dimer (HL) formed by an H chain associated through disulfide bridges with an L chain, as noted above. A divalent antibody is a tetramer formed by two HL dimers associated through at least one disulfide bridge. A polyvalent antibody is based on an aggregation of chains.

Of course, other recombinant antibodies composed of different sections of the antibodies of the invention are within the invention. In particular, the heavy chain constant region can be an IgG2, IgG3, IgG4, IgM or IgA antibody.

Another aspect of the monoclonal antibody according to the present invention then relates to an antibody, wherein said monoclonal antibody or antigen-binding fragment thereof is conjugated to a detectable label. Labels suitable for use in detection of a complex between an epitope and an antibody or antigen-binding fragment of the invention include, for example, a radioisotope, an epitope label (tag), an affinity label (e.g., biotin, avidin), a spin label, an enzyme, a toxin, or a fluorescent group or a chemiluminescent group.

In another aspect of the invention, the invention also encompasses an isolated nucleic acid encoding for monoclonal antibody or a fragment thereof as described above. The nucleic acid can be selected from RNA, DNA, PNA, and/or cDNA. The nucleic acid(s) of the invention can be isolated and/or cloned in vectors, such as plasmids, for example expression vectors. Thus, in addition to the protein fragments and regions of the antibodies, the present invention also encompasses the DNA sequence of the gene coding for the antibodies as well as the peptides encoded by said DNA. Particularly preferred DNA and peptide sequences are those relating to the variable regions of both the heavy and light chains of preferred antibodies, including the Complementarity Determining Regions ("CDR"), the hypervariable amino acid sequences within antibody variable regions which interact with amino acids on the complementary antigen. The invention includes these DNA and peptide sequences as well as DNA and peptide sequences that are homologous (share identity) to these sequences. In a preferred embodiment, these sequences are 80 % homologous although other preferred embodiments include sequences that are 85%, 90%, and 95% homologous. Determining these levels of homology for both the DNA and peptide sequence is well within the routine skill of those in the art.

The DNA sequences of the invention can be identified, isolated, cloned, and transferred to a prokaryotic or eukaryotic cell for expression by procedures well-known in the art. Such procedures are generally described in Sambrook et al., supra, as well as Current Protocols in Molecular Biology (Ausubel et al., eds., John Wiley & Sons), incorporated by reference.

In addition, the DNA and peptide sequences of the antibodies of the invention, including both monoclonal and chimeric antibodies, may form the basis of antibody "derivatives", which include, for example, the proteins or peptides encoded by truncated or modified genes. Such proteins or peptides may function similarly to the antibodies of the invention.

In another aspect of the invention, the invention also encompasses a hybridoma cell line expressing a monoclonal antibody according to the present invention, wherein said monoclonal antibody is produced by the hybridoma cell line huDkk3-16.1 (DSM ACC3274), huDkk3-2.13 (DSM ACC3275) or huDkk3-24.20 (DSM ACC3276) as deposited under the Budapest Treaty on September 8, 2015 at the DSMZ in Braunschweig, Germany, under the deposit numbers as indicated. Generally, a hybridoma is produced by fusing a suitable immortal cell line (e.g., a myeloma cell line) with antibody-producing cells. Antibody-producing cells can be obtained from the peripheral blood or, preferably the spleen or lymph nodes, of humans or other suitable animals immunized with the antigen of interest. The fused cells (hybridomas) can be isolated using selective culture conditions, and cloned by limiting dilution. Cells that produce antibodies with the desired specificity can be selected by a suitable assay (e.g., ELISA).

The present invention also discloses a pharmaceutical (e.g. diagnostic) composition comprising an antibody of the invention, monoclonal or chimeric, as well as fragments, regions, and derivatives thereof, together with a pharmaceutically acceptable carrier.

In another aspect thereof, the object of the present invention is solved by providing a method for diagnosing renal IF/TA or CKD, respectively, comprising the steps of a) obtaining a urine sample from a mammalian patient to be diagnosed, b) detecting the amount of Dickkopf 3 (Dkk3) protein and/or adducts thereof in said sample, and c) concluding on the extent of renal IF/TA or CKD of said patient, wherein a higher amount of Dkk3 protein and/or adducts thereof in said sample compared to the amount as detected in a urine sample of a healthy patient is indicative for higher grade renal IF/TA, i.e. more severe renal tissue damage, or progressive CKD, respectively.

Preferred is a method according to the present invention, wherein said detecting comprises the use of the monoclonal antibody or fragment thereof according to the invention.

In yet another aspect thereof, the object of the present invention is solved by providing a method as above, further comprising concluding on the progress of said renal IF/TA or CKD, respectively in said patient, wherein in step c) a higher amount of Dkk3 protein and/or adducts thereof in said sample compared to an amount in an earlier sample from said patient is indicative for progressive renal IF/TA or progressive CKD, respectively.

Preferred is a method according to the present invention, wherein said detecting comprises the use of the monoclonal antibody or fragment thereof according to the invention.

The mammalian patient can be a rat, mouse, goat, rabbit, sheep, horse, monkey, cat, or human, preferred is a mouse, rat, cat or human.

In the context of the experiments leading to the present invention, it was surprisingly found that Dkk3 protein as it is found in the urine can be a potent indicator for kidney failure and in particular for the presence of renal IF/TA.

In the context of the present invention, an "adduct" of the Dkk3 protein shall mean a degradation product of Dkk3 in the sample that, nevertheless, can be detected/is detected in the context of the present method. Adducts can be formed due to a reduced stability of the protein to be detected (here: Dkk3) at the temperatures as present during sampling, shipping, storage, and/or analysis. Adducts may be formed by protease activities as present in the biological sample as taken from the patient to be diagnosed. Furthermore, also covalent protein adducts formed after exposure to xenobiotics can be included in the analysis.

The Dkk3 gene is transcribed into three different isoforms (NM_015881, 2650 bp, NM_013253, 2635 bp, and NM_001018057, 2587 bp). Two of them result from alternative use of first exon (i.e. exon 1a and exon 1b, although they are both non-coding). One more variant lacks exon 1. All the variants share exons 2 to 8, and code for a 350 aa functioning protein. As used herein, the term "Dkk3" shall also include these isoforms.

The present inventors established two animal models, a mechanical and a toxic one, in which kidney failure was established. Both pathophysiological conditions reflect human diseases, namely:
- The unilateral ureteral obstruction (UUO) model; it is induced in mice by unilateral ureteral ligation of the left ureter. The animals rapidly develop, within a few days, severe renal IF/TA with an inflammatory cell infiltrate in the affected kidney. An obstruction of the ureter can be found also in mammals, such as human children and adults.
- The adenine diet model (ADM); high-adenine feeding in mice results in the formation of crystals in the renal tubules, with subsequent tubular injury and inflammation, obstruction, and finally obliteration (atrophy), always accompanied by marked interstitial fibrosis. These pathophysiological changes can also be found in mammals such as humans, both in children and adults. (For further discussion of animal models, see for example Neven E & D'Haese PC. Vascular calcification in chronic renal failure - what have we learned from animal studies? Circulation Research 2011; 108: 249-264).

WO 2010/132676 relates to methods and compositions for diagnosing CKD. WO 2010/132676 generally seeks to provide a diagnosis of CKD based on "panels" (i.e. several) of biomarkers as identified using expression analysis (i.e. "predictive expression signatures"). One of the biomarkers as mentioned is Dkk3, which is measured in a panel, and such measurement - allegedly - can be performed in a biological sample such as blood, urine, saliva, gastric juices, and the like. WO 2010/132676 did not perform an analysis using urine, and no problems with this analysis are mentioned. Instead, expression analysis on a chip based on renal tissue total RNA (examples 2-5), and histological samples were used (example 6).

Upon a more detail analysis of WO 2010/132676, the document does not contain any information that would qualify it as a promising springboard for developing a urine-based test for Dkk3 in the context of renal IF/TA as follows. For example, WO 2010/132676 uses a plurality of markers, an indication that no individually effective markers were identified. WO 2010/132676 then mentions "significant" expression changes, but this must not be confused with overall significance for the diagnosis. Also, WO 2010/132676 is silent with respect to problems with commonly available antibodies when detecting Dkk3.

Thus, according to the present invention, the expression of Dkk3 and its amount and/or concentration in the urine shows a clear correlation with renal tissue injury, specifically for renal IF/TA in a mammalian, preferably a human, patient.

Using wild-type mice instead of transgenic mice as used in WO 2010/132676, the inventors could furthermore show that - under conditions of stress in the kidney - Dkk3 is not expressed in the glomeruli, but solely in the epithelium of the tubules. These epithelia provide more than 90% of the cells in the kidney and have a direct contact to the urinary system.

Preferred is the method according to the present invention, wherein said patient to be diagnosed is a child or adolescent. In the context of the present invention, urine samples taken from a cohort of children with glomerular disease (glomerulonephritis) and primary tubular disease (nephronopthysis) were analyzed. Moreover, urine samples from a cohort of adult patients with different kidney diseases, that have undergone kidney biopsy, were analyzed.

For measuring and/or a detection of the mammalian such as human Dkk3 and/or adducts thereof in the sample, standard methods for assessing protein expression can be used, such as, for example, a method selected from the group of mass spectrometry, chromatography, SDS gel electrophoresis, and antigen/antibody reactions, such as, for example, Western blots and/or Enzyme-Linked Immunosorbent Assay (ELISA). Preferred is ELISA.

For measuring/detecting Dkk3 protein in urine samples, as explained herein, newly produced monoclonal antibodies according to the invention against recombinant human Dkk3 (huDkk3) were used in order to detect DKK3-epitopes, preferred are the methods according to the present invention, wherein said antigen/antibody reaction comprises the use of monoclonal antibodies and/or fragments thereof (such as Fab or scFv, and the like) that are specific for mammalian, such as human, Dkk3 and/or adducts thereof. Preferred is a method according to the present invention, wherein said detecting comprises the use of the monoclonal antibody or fragment thereof according to the invention.

Preferred is the method according to the present invention, wherein said progressive CKD are characterized by renal IF/TA. Renal IF can be defined as the accumulation of abnormal amounts of collagen and related molecules in the interstitium of the cortex, which serve as structural scaffolding. Renal TA can be an inevitable consequence of the chronic occlusion of the ureter.

Yet another aspect of the present invention then relates to a method for monitoring the progress of renal IF/TA or CKD, respectively, comprising the steps of a) obtaining a urine sample from a mammal, preferably a human, patient to be monitored having renal IF/TA or CKD, respectively, b) detecting the amount of Dkk3 protein and/or adducts thereof in said sample, and c) concluding on the progress of said renal IF/TA or CKD, respectively of said patient, wherein a higher amount of Dkk3 protein and/or adducts thereof in said sample compared to an earlier sample from said patient is indicative for progressing renal IF/TA or CKD, respectively. Vice versa, the detection of declining amounts of Dkk3 in the urine of said patient compared to an earlier sample from said patient would be a sign of diminishing the process of renal IF/TA, thus stopping the progression of CKD. Preferred is a method according to the present invention, wherein said patient having renal IF/TA or CKD, respectively has been diagnosed using a method according to the present invention as above. Preferred is a method according to the present invention, wherein said detecting comprises the use of the monoclonal antibody or fragment thereof according to the invention.

Preferred is the method for monitoring according to the present invention, wherein said sample consists or consists essentially of urine, either from a patient to be diagnosed or from a group of patients, such as, for example, a group of children (i.e. a pooled sample). Preferred is the method for monitoring according to the present invention, wherein said patient to be diagnosed is a child or adolescent.

For measuring and/or a detection of the mammalian, such as human, Dkk3 and/or adducts thereof in the sample, also in the context of the monitoring standard methods for assessing protein expression can be used, such as, for example, a method selected from the group of mass spectrometry, chromatography, SDS gel electrophoresis, and antigen/antibody reactions, such as, for example, Western blots and/or Enzyme-Linked Immunosorbent Assay (ELISA). Preferred is ELISA. Preferred is a method according to the present invention, wherein said detecting comprises the use of the monoclonal antibody or fragment thereof according to the invention.

Preferred is a method for monitoring according to the present invention, wherein said patient is undergoing treatment for renal IF/TA respectively CKD. Said treatment can preferably be performed as described below.

In addition to the above approaches, new targets for the therapy of renal IF/TA respectively CKD are desired. Thus, according to another aspect thereof, the present invention provides a method for detecting and/or identifying a compound suitable for the treatment of progressive renal IF/TA or CKD, respectively, comprising the steps of a) administering a candidate compound to a mammal having progressive renal IF/TA respectively CKD, b) detecting the amount of Dickkopf 3 (Dkk3) protein and/or adducts thereof in a urine sample as obtained from said mammal, and c) identifying a compound suitable for the treatment of said progressive renal IF/TA or CKD, respectively, in said mammal, wherein a lower amount of Dkk3 protein and/or adducts thereof in said sample compared to the amount as detected in an earlier sample from said mammal is indicative for a compound suitable for the treatment of said progressive renal IF/TA or CKD, respectively. Said mammal can be a rat, mouse, goat, rabbit, sheep, horse, monkey, cat or human, preferred is a mouse, rat, cat or human.

Preferred is the method for detecting and/or identifying according to the present invention, wherein said sample consists or consists essentially of urine, either from a patient to be diagnosed or from a group of patients, such as, for example, a group of children (i.e. a pooled sample). Preferred is the method for detecting and/or identifying according to the present invention, wherein said patient to be treated and/or diagnosed is a child or adolescent.

For measuring and/or a detection of the mammalian, such as human, Dkk3 and/or adducts thereof in the sample, also in the context of the detecting and/or identifying standard methods for assessing protein expression can be used, such as, for example, a method selected from the group of mass spectrometry, chromatography, SDS gel electrophoresis, and antigen/antibody reactions, such as, for example, Western blots and/or Enzyme-Linked Immunosorbent Assay (ELISA). Preferred is ELISA. Preferred is a method according to the present invention, wherein said detecting comprises the use of the monoclonal antibody or fragment thereof according to the invention.

More preferred is a method for detecting and/or identifying according to the present invention, wherein said compound is selected from the group consisting of a peptide library, a combinatory library, a cell extract, in particular a plant cell extract, a "small molecular drug", an antisense oligonucleotide, an siRNA, an mRNA and an antibody or fragment thereof (such as Fab or scFv, and the like).

The method according to the present invention as described herein is thus suitable for the identification of compounds that can modulate the expression of Dkk3 in a cell/in cells. The compound may also be the monoclonal antibody or fragment thereof according to the invention.

Preferred is a method for detecting and/or identifying according to the present invention, further comprising testing said compound(s) as detected/identified for its activity on renal IF/TA or CKD, respectively. Respective assays are known to the person of skill, and can be taken from the respective literature.

Preferred is a method for detecting and/or identifying according to the present invention, wherein steps a) to c) are repeated, and, optionally, chemically modifying said compound before said repeating. The thus identified candidate compound can then, in a preferred embodiment, modified in a further step. Modification can be effected by a variety of methods known in the art, which include, without limitation, the introduction of novel side chains or the exchange of functional groups like, for example, introduction of halogens, in particular F, Cl or Br, the introduction of lower alkyl groups, preferably having one to five carbon atoms like, for example, methyl, ethyl, n-propyl, isopropyl, n-butyl, isobutyl, tertbutyl, n-pentyl or isopentyl groups, lower alkenyl groups, preferably having two to five carbon atoms, lower alynyl groups, preferably having two to five carbon atoms or through the introduction of, for example, a group selected from the group consisting of NH₂, NO₂, OH, SH, NH, CN, aryl, heteroaryl, COH or COOH group. The thus modified binding substances are than individually tested with a method of the present invention. If needed, the steps of selecting the candidate compound, modifying the compound, and testing compound can be repeated a third or any given number of times as required. The above described method is also termed "directed evolution" since it involves a multitude of steps including modification and selection, whereby binding compounds are selected in an "evolutionary" process optimizing its capabilities with respect to a particular property, e.g. its ability to modulate the expression of the Dkk3 polypeptide.

Preferred is a method for detecting and/or identifying according to the present invention, wherein said patient is undergoing treatment for renal IF/TA or CKD, respectively. Said treatment can preferably be performed as described below.

Another aspect of the present invention relates to a method for manufacturing a pharmaceutical composition for treating or preventing renal IF/TA or CKD, respectively, comprising the steps of: performing a method for detecting and/or identifying according to the present invention, and formulating said compound as detected and identified into a pharmaceutical composition.

In a further embodiment of the method of the present invention, the compound identified as outlined above, which may or may not have gone through additional rounds of modification and selection, is admixed with suitable auxiliary substances and/or additives. Such substances comprise pharmacological acceptable substances, which increase the stability, solubility, biocompatibility, or biological half-life of the interacting compound or comprise substances or materials, which have to be included for certain routes of application like, for example, intravenous solution, sprays, band-aids or pills.

Carriers, excipients and strategies to formulate a pharmaceutical composition, for example to be administered systemically or topically, by any conventional route, in particular enterally, e.g. orally, e.g. in the form of tablets or capsules, parenterally, e.g. in the form of injectable solutions or suspensions, topically, e.g. in the form of lotions, gels, ointments or creams, or in nasal or a suppository form are well known to the person of skill and described in the respective literature.

Administration of an agent, e.g., a compound can be accomplished by any method which allows the agent to reach the target cells. These methods include, e.g., injection, deposition, implantation, suppositories, oral ingestion, inhalation, topical administration, or any other method of administration where access to the target cells by the agent is obtained. Injections can be, e.g., intravenous, intradermal, subcutaneous, intramuscular or intraperitoneal. Implantation includes inserting implantable drug delivery systems, e.g., microspheres, hydrogels, polymeric reservoirs, cholesterol matrices, polymeric systems, e.g., matrix erosion and/or diffusion systems and non-polymeric systems, e.g., compressed, fused or partially fused pellets. Suppositories include glycerin suppositories. Oral ingestion doses can be enterically coated. Inhalation includes administering the agent with an aerosol in an inhalator, either alone or attached to a carrier that can be absorbed. The agent can be suspended in liquid, e.g., in dissolved or colloidal form. The liquid can be a solvent, partial solvent or non-solvent. In many cases, water or an organic liquid can be used. The compound may also be the monoclonal antibody or fragment thereof according to the invention.

Yet another aspect of the present invention is directed at a pharmaceutical composition for treating or preventing renal IF/TA or CKD, respectively, obtainable or obtained by a method according to the method as above.

In certain embodiments, the compound is administered to the subject by administering a recombinant nucleic acid, such as, for example, an anti-Dkk3 RNA, for example an si-RNA. Preferably, the recombinant nucleic acid is a gene therapy vector.

Another aspect of the present invention relates to a method or use as described herein, wherein the pharmaceutical composition further comprises additional pharmaceutically active ingredients for treating renal IF/TA or CKD, respectively, i.e. chemotherapeutics.

Another aspect of the present invention then relates to a method for treating or preventing renal IF/TA or CKD, respectively, in a mammal, such as human, patient, comprising administering to said patient an effective amount of a pharmaceutical composition according to the invention as above. In general, the attending physician will base a treatment on the compound as identified, and optionally also on other individual patient data (clinical data, family history, DNA, etc.), and a treatment can also be performed based on the combination of these factors. This method of the present invention for example involves integrating individual diagnostic kidney disease data with patient clinical information and general healthcare statistics to enable, for example, the application of personalized medicine to the patient. Significant information about drug effectiveness, drug interactions, and other patient status conditions can be used,too.

Preferred is a therapeutic method according to the present invention, wherein said mammal to be treated is a rat, mouse, goat, rabbit, sheep, horse, monkey, cat or human, preferred is a mouse, rat, cat or human, such as a child, adolescent or adult. Treatment is meant to include, e.g., preventing, treating, reducing the symptoms of, or curing the disease or condition, i.e. renal IF/TA or CKD, respectively.

An "effective amount" is an amount of the compound(s) or the pharmaceutical composition as described herein that reduces on the expression and/or abundance of Dkk3 in kidney cells and/or urine. The amount alleviates symptoms as found for renal IF/TA or CKD, respectively. Alleviating is meant to include, e.g., preventing, treating, reducing the symptoms of, or curing the disease (renal IF/TA or CKD, respectively) or condition. The compound may also be the monoclonal antibody or fragment thereof according to the invention.

The invention also includes a method for treating a subject at risk for renal IF/TA or CKD, respectively, and/or progression of the disease, wherein a therapeutically effective amount of a compound as above is provided. Being at risk for the disease can result from, e.g., a family history of the disease, a genotype which predisposes to the disease, or phenotypic symptoms which predispose to the disease. A further aspect of the present invention is the use of a modulator of the expression of Dkk3 for the manufacture of a pharmaceutical composition for treating or preventing renal IF/TA or CKD, respectively. Preferably, said modulator is an inhibitor of the expression of Dkk3 in the kidney as described herein.

Yet another preferred aspect of the present invention then relates to the use of a diagnostic kit, comprising materials for diagnosing and/or monitoring renal IF/TA or CKD, respectively, in a human patient in a method according to the present invention as described herein, in one or separate containers, preferably comprising materials for measuring the amount of Dkk3 protein and/or adducts thereof in a human urine sample. The materials may comprise the monoclonal antibody or fragment thereof according to the invention. Optionally, the kit comprises instructions for performing a method according to the present invention as described herein.

The kit may further comprise one or more of (iii) a buffer, (iv) a diluent, (v) a filter, (vi) a needle, or (v) a syringe. The container is preferably a bottle, a vial, a syringe or test tube; and it may be a multi-use container. The container may be formed from a variety of materials such as glass or plastic. Preferably the kit and/or container contain/s instructions on or associated with the container that indicates directions for reconstitution and/or use.

Preferred is the use according to the present invention, wherein said kit comprises materials for a method selected from the group of Western blots and/or Enzyme-Linked Immunosorbent Assay (ELISA). For example, the label may indicate that the lyophilized formulation is to be reconstituted to certain antibody concentrations as suitable for the above methods, such as ELISA.

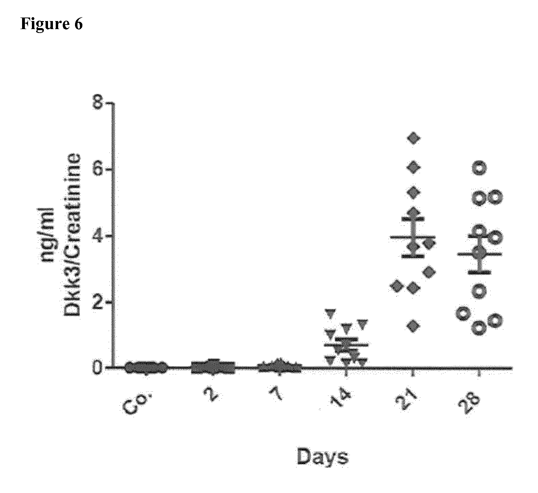

Further preferred is the use according to the present invention, wherein said kit comprises monoclonal antibodies or fragments thereof specific for human Dkk3 and/or adducts thereof according to the invention (see also Figure 6).

The present invention shall now be further described in the following examples with reference to the accompanying figures, nevertheless, without being limited thereto. All references as cited herein are incorporated by reference in their entities.
**Figure 1** shows an overview of the risk of progression of CKD according to: Kidney Disease Improving Global Outcomes (KDIGO) 2012 Clinical Practice Guideline for the Evaluation and Management of Chronic Kidney Disease. The categorization is based on glomerular filtration rate (GFR) and urine albumin excretion rate (albuminuria). In: Stevens PE & Levin A; Kidney Disease: Improving Global Outcomes Chronic Kidney Disease Guideline Development Work Group Members. Evaluation and management of chronic kidney disease: synopsis of the kidney disease: improving global outcomes 2012 clinical practice guideline. Annals of Internal Medicine 2013; 158: 825-830.
**Figure 2** shows the prevalence of CKD stages by age group in the National Health and Nutrition Examination Survey (NHANES) 1988-1994 and 1999-2004. In: Coresh J, Selvin E, Stevens LA, Manzi J, Kusek JW, Eggers P, Van Lente F, Levey AS. Prevalence of chronic kidney disease in the United States. JAMA 2007; 298: 2038-2047.
**Figure 3** summarizes the distribution of causes of CKD worldwide. CGN = chronic glomerulonephritis, HT = hypertensive nephrosclerosis, CIN = chronic interstitial nephritis, RVD = renovascular disease. In: Jha V, Garcia-Garcia G, Iseki K, Li Z, Naicker S, Plattner B, Saran R, Wang AY, Yang CW. Chronic kidney disease: global dimension and perspectives. Lancet 2013; 382: 260-272.

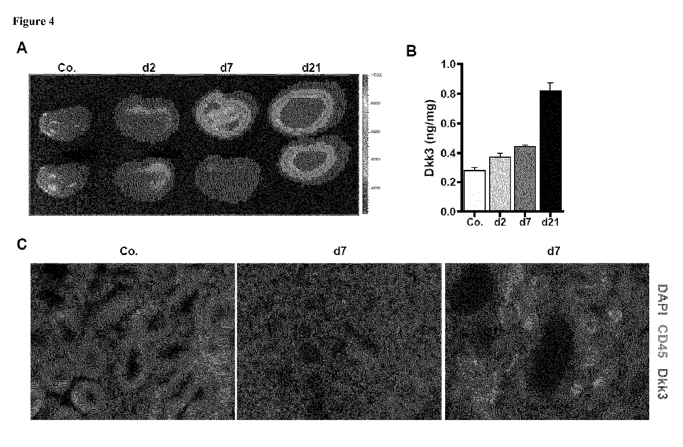

**Figure 4** shows that renal Dkk3 production is induced in tubular epithelial cells during development of interstitial fibrosis. (A+D) Ex vivo bioluminescence imaging of fibrotic kidneys of Dkk3-LCh mice (A) 2, 7 and 21 days after induction of unilateral ureteral obstruction (UUO). As a control, kidneys from untreated Dkk3-LCh mice were used. 5 minutes after i.p. injection of 150 mg/kg D-luciferin, mice were sacrificed and organs were taken out. After 5 minutes of incubation in a 1 mg/ml D-luciferin solution in PBS at 37°C organs were imaged for 5 minutes. Colors display intensity of the emitted light (see scale). One representative experiment out of 3 is shown. (B+E) Amount of Dkk3 protein relative to organ weight in lysates of fibrotic kidneys (B) 2, 7 and 21 days after induction of UUO or (E) 2, 7 and 28 days after starting of adenine feeding. As a control, kidneys from untreated mice were used. (C) Detection of mCherry expression in fibrotic kidneys of Dkk3-LCh mice 7 days after UUO induction. As a control kidneys of untreated Dkk3-LCh mice were used. Cryo sections from isolated kidneys were prepared and fluorochrome conjugated anti-mCherry staining (red) and anti-CD45 staining (green) was performed. Nuclei were counter stained with DAPI (blue). Shape and localization of mCherry positive cells identified them as tubular epithelial cells. One representative experiment out of three is shown.

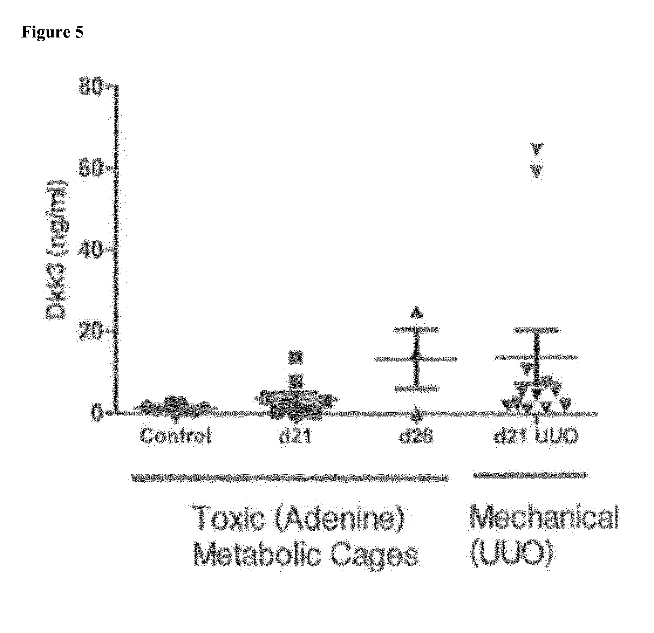

**Figure 5** shows the failure to reliably detect Dkk3 in pooled mice urine samples using the model of metabolic cages.
**Figure 6** shows that urine was found to be suitable for a significant detection of Dkk3; it shows the detection of Dkk3 in the urine of mice in the model of the adenine-diet fed mouse. A clear increase of the quotient between creatinine and Dkk3 was found, starting after 7 days.

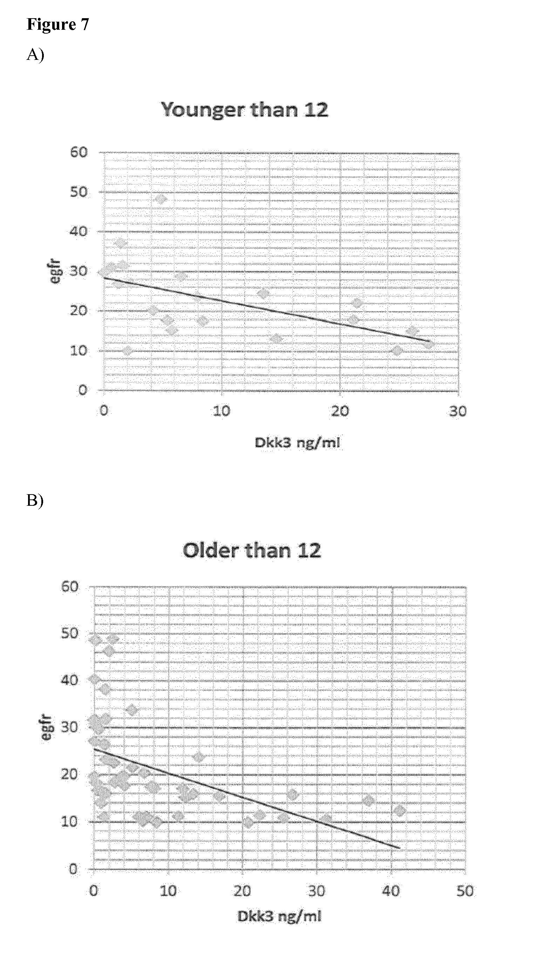

**Figure 7** shows measurements on urine samples derived from children having CKD, such as nephronophthisis versus samples from adults (B), showing an advantage of the assays according to the present invention for children. eGFR = estimated glomerular filtration rate; R2 for younger than 12 = 0.30; R2 for older than 12 = 0.24

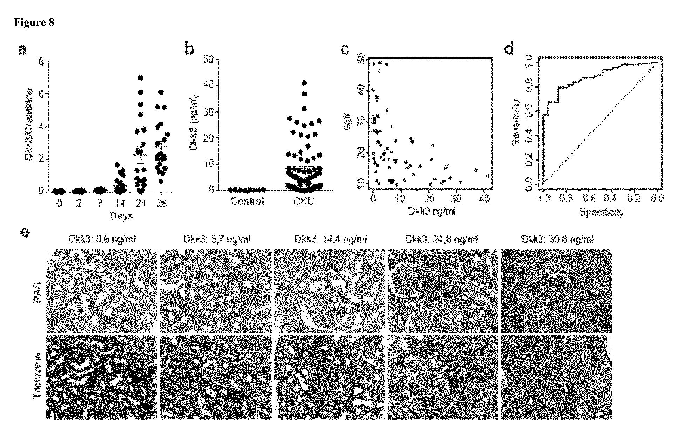

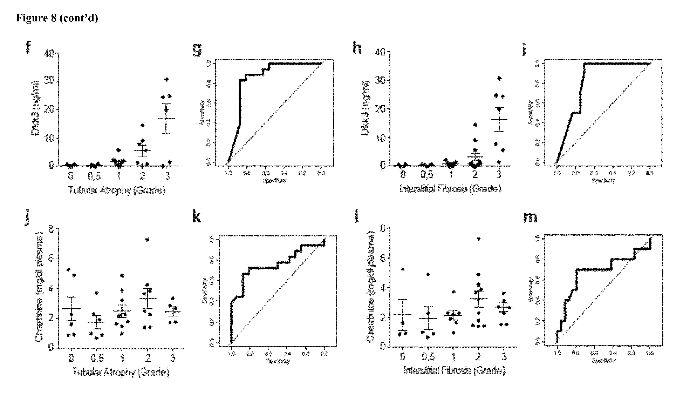

**Figure 8** shows that Dkk3 in the urine of patients with different types of CKD is a marker for renal IF/TA (see also example 4). (a) Dkk3 level detected in the urine of mice fed with adenine-enriched diet for 2, 7, 14, 21, and 28 days. As control, urine from mice before the beginning of the adenine administration has been taken. (b) Dkk3 level detected in the urine of healthy (control) young volunteers and patients with CKD. (c) Correlation between eGFR and Dkk3 urine levels observed in pediatric patient samples (d) ROC curve derived for Dkk3, using a threshold of 25 mL/min/1.73 m² for the estimated glomerular filtration rate (eGFR) and defining the two patient groups as those with eGFR ≤25 having severe disease, and those with eGFR >25 having non-severe disease. AUC=0.88 (95% confidence interval: 0.81-0.96). Sensitivity (at 90% specificity) is 0.71 (95% CI 0.51-0.90). Specificity (at 90% sensitivity) is 0.52 (95% CI 0.26-0.83). (e) Representative PAS- and trichrome-stained section images of kidney biopsies from adult patients with CKD and related urine Dkk3 concentrations. (f) Correlation between Dkk3 urine levels and degree of tubular atrophy (TA) in adult patient kidney biopsy samples. (g) ROC curve derived for Dkk3, using a threshold of 25% tubular atrophy area. AUC=0.87 (95% CI 0.73-1.00). (h) Correlation between Dkk3 urine levels and degree of interstitial fibrosis (IF) in adult patient kidney biopsy samples. (i) ROC curve derived for Dkk3, using a threshold of 25% interstitial fibrosis area. AUC=0.83 (95% CI 0.69-0.96). (j) Correlation between creatinine blood levels and degree of tubular atrophy (TA) in adult patient kidney biopsy samples. (k) ROC curve derived for creatinine, using a threshold of 25% tubular atrophy area. AUC=0.76 (95% CI 0.59-0.93). (1) Correlation between creatinine blood levels and degree of interstitial fibrosis (IF) in adult patient kidney biopsy samples. (m) ROC curve derived for creatinine, using a threshold of 25% interstitial fibrosis area. AUC=0.68 (95% CI 0.45-0.92).

### Examples

### Example 1: Production of preferred effective monoclonal antibodies against recombinant human Dickkopf 3 (huDkk3) protein

Female Dickkopf 3 knock-out (Dkk3^{-/-}) mice were immunized several times subcutaneously with purified human Dkk3-mouseIgG2b fusion protein. Three days after the last boost spleen cells were fused with the myeloma cell line X63-Ag8.653 using polyethylene glycol 4000. Supernatants of growing hybridomas were screened by ELISA for reactivity with recombinant human Dkk3 (huDkk3) protein. Thus, hybridomas huDkk3-2.13 (IgG1 kappa isotype), huDkk3-16.1 (IgG1 kappa isotype) and huDkk3-24.20 (IgG1 kappa isotype) were identified and cloned by limiting dilution.

Antibody mass production was performed in a MiniPERM modular bioreactor (Greiner, Frickenhausen, Germany) and immunoglobulin was purified by affinity chromatography over protein A Sepharose CL-4B (GE Healthcare, Freiburg, Germany). Specificity of purified antibodies was verified by Western blotting of lysates derived from HEK293T huDkk3 transfectants or mock transfected HEK293T cells and intracellular flow cytometry using the same cell lines. As revealed by competitive binding inhibition assay, the three monoclonal antibodies recognize different epitopes on the huDkk3 molecule.

The hybridoma cell lines huDkk3-2.13, huDkk3-16.1, and huDkk3-24.20 were deposited (depositing entity: Deutsches Krebsforschungszentrum (DKFZ) - Stiftung öffentlichen Rechts, Heidelberg, Germany) at the Leibniz-Institut DSMZ-Deutsche Sammlung von Mikroorganismen und Zellkulturen GmbH in 38124 Braunschweig, Germany on September 8, 2015 under the depositing numbers DSM ACC3275, DSM ACC3274, and DSM ACC3276, respectively.

### Example 2: Establishing an enzyme-linked immunosorbent assay (ELISA) for the improved detection of huDkk3 in urine

In a preferred method, for the analysis of huDkk3 protein, human urine samples were tested using an ELISA-based method. Micro Test III Flexible Assay Plate (BD Falcon #353912, BD Biosciences, USA) was used as a sample plate.

Although other Dkk3 human ELISA kits (e.g. from Creative Diagnostics, USA; or from antibodies-online Inc., USA) could be used, it was found that the sensitivity and the specificity of the newly produced recombinant huDkk 3 monoclonal antibodies (see Example 1) were superior after adjustment to the conditions in urine samples. Urine samples were either used undiluted or samples were further diluted in PBS-Tween.

As mentioned already, although other Dkk3 human ELISA kits (e.g. from Creative Diagnostics, USA; or from antibodies-online Inc., USA) are available, the newly produced huDkk three monoclonal antibodies have superior properties, because they provide a 100% reproducibility in the assay(s), and thereby avoid variations in sensitivity and specificity, as they are introduced, e.g. by the use of antisera from different animals. Furthermore, the combined use of 2 or all 3 of the antibodies even further improves the assays, as they were found to recognize (bind to) different epitopes of DKK3, rendering the assay even more robust, in particular in a high-throughput environment.

### Buffers

PBS pH 7.2
8.1 mM Na₂HPO₄ x 2H₂O
1.5 mM K₂H₂PO₄
2.6 mM KCl
137 mM NaCl

PBS-Tween
PBS + 0.05 % Tween 20

Substrate buffer pH 6.0
0.1 M KH₂PO₄

0.2 % gelatin in PBS with 0.1 % NaN₃
2 M H₂SO₄

| | |
|---|---|
| Coating: | Monoclonal antibody huDkk3-16.1 (1 mg/ml) |
| | |
| Conjugates: | Biotinylated monoclonal antibodies huDkk3-2.13 and huDkk3-24.20 (1 mg/ml) |
| | |
| | Streptavidin-peroxidase (#016-030-084, Dianova) |
| Substrate: | Orthophenylendiamine (OPD, Sigma); H₂O₂ (30%) (Perhydrol, Merck) |
| | |
| Calibration protein: | Recombinant human Dkk3 protein (1118-DK; R&D Systems, USA) |
| | |
| Equipment: | Titertek 8-channel pipette |
| | Eppendorf-pipettes and tips |
| | Vaccu-Pette (96-channel pipette for microtiter plates, Neo-Lab) |
| | Titertek Multiscan Plus MKII (ELISA photometer) |

For coating, the plate was incubated over night at 4 °C with 100 ng monoclonal antibody huDkk3-16.1 in PBS in 100 µl per well.

The plates were then washed by reversing the plates and applying about 200 µl of PBS-Tween (0.05 % Tween 20 in PBS). After the second washing step, as much liquid as possible was removed. For blocking, 200 µl of 0.2 % gelatin in PBS plus 0.1 % sodium azide was filled into each well, followed by incubation for 1 hour at 37 °C. The coated plates were stored until use at 4 °C (storage is possible for several months, but drying has to be prevented).

For positive control, serial dilutions of recombinant huDkk3 protein were employed in order to prepare a calibration (standard) curve. Samples were prepared in PBS Tween, 100 µl were added into each well.

| | |
|---|---|
| Calibration protein: | recombinant huDkk3; serial dilution from 0.5 to 32 ng/ml |
| Test samples: | four dilutions 1:50; 1:100; 1:500; and 1:1000 |
| Incubation: | 1 hour at room temperature, followed by 4 times washing using PBS-Tween as described above |

After removal of blocking buffer, urine samples were pipetted into the assay plate at a volume of 100 µl/well and incubated 1 hour at room temperature.

Plates were washed 4 times with PBS-Tween and 100 µl/well of a 1:1 mixture ofbiotinylated monoclonal antibodies huDkk3-2.13 and huDkk3-24.20 at a final concentration of 2 µg/ml each - was added for another 1 hour at room temperature. Following 4 washes 100 µl/well streptavidin-peroxidase diluted 1:2000 in PBS-Tween was added for 30 minutes at room temperature. After another 4 washing cycles a substrate reaction was obtained by weighing in orthophenylenediamine (OPD) with complete dissolution in 100 mM sodium hydrogen phosphate buffer (at a concentration of 1 mg/ml) containing 0.03 % peroxide (H₂O₂). After subsequent shaking 100 µl of substrate solution were quickly added to each well. The plate was covered in order to protect the reactions from light.

The color reaction was stopped after approximately 1-10 minutes by adding 50 µl of 2 M sulphuric acid (H₂SO₄) per well.

Finally, the reactions were measured using photometric analysis in a Multiscan Plus MKII photometer with optical densities at 492 nm (OD₄₉₂). First, the calibration curve for huDkk3 was generated. All sample concentrations were determined with reference to said curve, and the actual protein concentration was calculated by multiplication with the respective dilution factor.

### Example 3: Expression analysis of Dkk3 in a mouse model

Using standard transgenic technology in mice, reporter mice were produced that contained transgenic Dkk3-constructs, where the Dkk3-promoter was linked to the marker protein green-fluorescence protein (GFP) that provides a fluorescent signal when expressed. Since the physiology of the kidney in mice is comparable with the human kidneys, it is expected that the expression in mice is identical to the one in human. It was found that Dkk3 was exclusively expressed in the epithelium of the renal tubuli (see Figure 4).

### Example 4: Analysis of Dkk3 in the urine of adults with kidney diseases

Using the above-described assay, significant amounts of Dkk3 were detected in the urine of adults having different types of kidney disease and undergoing kidney biopsy. The results as depicted in the following Table 1 showing an increase of Dkk3 in urine of patients with higher percentage/grade of renal IF/TA, indicating more severe kidney injury. FSGS = focal-segmental sclerosis.

**Table 1: increase of Dkk3 in urine of patients with higher percentage/grade of renal IF/TA. FSGS = focal-segmental sclerosis.**

| **Sample#** | **Gender** | **Age** | **Diagnosis** | **Dkk3 (ng/ml)** | **Plasma Creatinine (mg/dl)** | **GFR (ml/ min/ 1,73 m²)** | **%T A** | **TA** | **%IF** | **IF** | **%IF TA** | **IFT** |
|---|---|---|---|---|---|---|---|---|---|---|---|---|
| 2 | female | 56 | Transplanted kidney | 0.6 | 0,92 | 70,3 | 0 | 0 | 4,9 | 0 | 2,45 | 0 |
| 4 | male | 78 | Thrombotic microangiopathy | 5,6 | 5,26 | 9,7 | 60 | 2 | 70 | 3 | 65 | 2.5 |
| 6 | male | 56 | Glomerulonephritis | 0 | 1,61 | 35,9 | 25 | 1 | 15 | 0,5 | 20 | 0,75 |
| 8 | male | 50 | Glomerulonephritis | 0 | 0,88 | 100, | 10 | 0,5 | 4,9 | 0 | 7.45 | 0,25 |
| 10 | female | 45 | Acute interstitial nephritis | 0 | 2,27 | 25,6 | 70 | 3 | 60 | 2 | 65 | 2,5 |
| 13 | female | 51 | Glomerulonephritis | 0 | 0,68 | 102 | 4,9 | 0 | 10 | 0,5 | 7,45 | 0.25 |
| 14 | male | 77 | FSGS | 0 | 0,99 | 73,8 | 25 | 1 | 35 | 1 | 30 | 1 |
| 16 | female | 79 | Transplanted kidney | 0,5 | 0,89 | 62,7 | 15 | 0.5 | 35 | 2 | 25 | 1,25 |
| 17 | male | 59 | Benign nephrosclerosis | 0,5 | 3,71 | 16,9 | 0 | 0 | 15 | 0,5 | 7,5 | 0.25 |
| 18 | male | 76 | Transplanted kidney | 0 | 1,91 | 35,3 | 40 | 2 | 50 | 2 | 45 | 2 |
| 21 | female | 55 | Glomerulonephritis | 0 | 0,97 | 87,8 | 0 | 0 | 4,9 | 0 | 2,45 | 0 |
| 22 | female | 60 | Glomerulonephritis | 14,4 | 2,21 | 31,6 | 40 | 2 | 50 | 2 | 45 | 2 |
| 27 | male | 73 | Transplanted kidney | 0.3 | 3,21 | 18,2 | 25 | 1 | 30 | 1 | 27.5 | 1 |
| 30 | male | 52 | FSGS | 24,8 | 3,88 | 15,8 | 80 | 3 | 80 | 3 | 80 | 3 |
| 42 | female | 75 | Transplanted kidney | 0 | 1,8 | 27,4 | 10 | 0,5 | 30 | 1 | 20 | 0,75 |
| 43 | male | 74 | Transplanted kidney | 0 | 1,47 | 46,9 | 15 | 0,5 | 20 | 1 | 17,5 | 0,75 |
| 49 | female | 39 | Thrombotic microangiopathy | 30,8 | 4,88 | 10,6 | 65 | 2 | 100 | 3 | 82,5 | 2,5 |
| 51 | male | 32 | Acute tubular damage | 0,4 | 7,28 | 11,9 | 0 | 0 | 0 | 0 | 0 | 0 |
| 57 | male | 22 | Transplanted kidney | 5,7 | 2,28 | 36,1 | 30 | 1 | 45 | 2 | 37,5 | 1,5 |
| 63 | male | 50 | Glomerulonephritis | 1,1 | 1,42 | 57,8 | 30 | 1 | 60 | 2 | 45 | 1,5 |
| 68 | male | 68 | FSGS | 0,4 | 1,39 | 40.6 | 5 | 0,5 | 15 | 0,5 | 10 | 0,5 |
| 69 | male | 48 | Transplanted kidney | 0,5 | 2,49 | 29.6 | 4,9 | 0 | 15 | 0,5 | 9.95 | 0,25 |
| 70 | male | 57 | Glomerulonephritis | 9,1 | 3,84 | 16,5 | 50 | 2 | 65 | 2 | 37,5 | 2 |
| 72 | male | 48 | FSGS | 24,4 | 3,35 | 20,8 | 80 | 3 | 80 | 3 | 80 | 3 |
| 73 | female | 41 | Transplanted kidney | 0.9 | 1,73 | 48,3 | 10 | 0,5 | 30 | 1 | 20 | 0,75 |
| 76 | female | 69 | Chronic interstitial nephritis | 1,4 | 2,69 | 17,6 | 70 | 3 | 75 | 3 | 72.5 | 3 |
| 78 | female | 67 | Glomerulonephritis | 0.3 | 2,89 | 21,7 | 20 | 1 | 35 | 2 | 27,5 | 1,5 |
| 83 | male | 49 | FSGS | 1,9 | 2,09 | 27,4 | 30 | 1 | 50 | 2 | 40 | 1,5 |
| 86 | female | 32 | FSGS | 1,1 | 1,84 | 47,9 | 35 | 2 | 50 | 2 | 42,5 | 2 |
| 88 | male | 49 | Glomerulonephritis | 1,5 | 0.78 | 89,7 | 20 | 1 | 30 | 1 | 25 | 1 |
| 90 | male | 70 | Glomerulonephritis | 20 | 2,5 | 14 | 70 | 3 | 80 | 3 | 75 | 3 |
| 98 | female | 75 | Transplanted kidney | 7,9 | 3.19 | 18,1 | 60 | 2 | 70 | 3 | 65 | 2,5 |
| 100 | male | 67 | Transplanted kidney | 0,7 | 1,39 | 30,7 | 35 | 2 | 35 | 2 | 35 | 2 |
| 104 | male | 74 | Glomerulonephritis | 2,2 | 1,89 | 34,5 | 25 | 1 | 40 | 1 | 32,5 | 1 |

## Claims

1. A monoclonal antibody specific for Dickkopf 3 (Dkk3) protein, comprising at least one light chain and at least one heavy chain, wherein said at least one light chain comprises a polypeptide comprising an amino acid sequence having at least 90% identity with a light chain variable region of a monoclonal antibody and wherein said at least one heavy chain comprises a polypeptide comprising an amino acid sequence having at least 90% identity with a heavy chain variable region of a monoclonal antibody, wherein said monoclonal antibody is as produced by the hybridoma cell line huDkk3-16.1, huDkk3-2.13 or huDkk3-24.20 as deposited under the Budapest Treaty on September 8, 2015 at the DSMZ in Braunschweig, Germany under deposit number DSM ACC3274, DSM ACC3275, and DSM ACC3276, respectively.

2. The monoclonal antibody according to claim 1, comprising a Fab, Fab', F(ab')2, Fv, SFv, or scFv.

3. The monoclonal antibody according to claim 1 or 2, wherein the percentages identity are at least 95%, for example, at least 99%, or 100%.

4. The monoclonal antibody according to any one of claims 1 to 3, wherein said monoclonal antibody or antigen-binding fragment thereof is conjugated or bound to at least one detectable label.

5. An isolated nucleic acid encoding for a monoclonal antibody or antigen-binding fragment thereof according to any of claims 1 to 3.

6. A hybridoma cell line expressing a monoclonal antibody according to claim 1, selected from the hybridoma cell lines huDkk3-16.1, huDkk3-2.13 and huDkk3-24.20 as deposited under the Budapest Treaty on September 8, 2015 at the DSMZ in Braunschweig, Germany under deposit number DSM ACC3274, DSM ACC3275, and DSM ACC3276, respectively.

7. A method for diagnosing renal IF/TA or CKD, respectively, comprising the steps of
a) detecting the amount of Dickkopf 3 (Dkk3) protein and/or adducts thereof in a urine sample obtained from a mammalian patient to be diagnosed, and
c) concluding on the extent of renal IF/TA or CKD of said patient,
wherein a higher amount of Dkk3 protein and/or adducts thereof in said sample compared to the amount as detected in a urine sample of a healthy patient is indicative for higher grade renal IF/TA or progressive CKD, respectively.

8. The method according to claim 7, further comprising concluding on the progress of said renal IF/TA or CKD, respectively in said patient, wherein in step c) a higher amount of Dkk3 protein and/or adducts thereof in said sample compared to an amount in an earlier sample from said patient is indicative for progressive renal IF/TA or progressive CKD, respectively.

9. The method according to claim 7 or 8, wherein said detecting comprises a method selected from the group of mass spectrometry, chromatography, SDS gel electrophoresis, and antigen/antibody reactions, such as, for example, Western blots and/or Enzyme-Linked Immunosorbent Assay (ELISA).

10. The method according to any of claims 7 to 9, wherein said detecting comprises the use of the monoclonal antibody or fragment thereof according to any one of claims 1 to 4.

11. The method according to any of claims 7 to 10, wherein said patient is a child or adolescent.

12. A method for identifying a compound suitable for the treatment of progressive renal IF/TA or CKD, respectively, comprising the steps of
a) administering a candidate compound to a mammal having progressive renal IF/TA or CKD, respectively
b) detecting the amount of Dickkopf 3 (Dkk3) protein and/or adducts thereof in a urine sample as obtained from said mammal, and
c) identifying a compound suitable for the treatment of said progressive renal IF/TA or CKD, respectively, in said mammal, wherein a lower amount of Dkk3 protein and/or adducts thereof in said sample compared to the amount as detected in an earlier sample from said mammal is indicative for a compound suitable for the treatment of said progressive renal IF/TA or CKD, respectively.

13. The method according to claim 12, wherein steps a) to c) are repeated, and, optionally, chemically modifying said compound before said repeating.

14. The method according to claim 12 or 13, wherein said detecting comprises the use of the monoclonal antibody or fragment thereof according to any one of claims 1 to 4.

15. Use of the monoclonal antibody or fragment thereof according to any one of claims 1 to 4 for detecting the amount of Dickkopf 3 (Dkk3) protein and/or adducts thereof in a mammalian urine sample for diagnosing and/or monitoring progressive renal IF/TA or CKD, respectively in a mammalian patient.
